# EUROPÄISCHE PATENTSCHRIFT

(11) **EP 2 361 067 B1**
(45) Veröffentlichungstag und Bekanntmachung des Hinweises auf die Patenterteilung: **15.08.2018**
(21) Anmeldenummer: 09796359.9
(22) Anmeldetag: 10.12.2009
(51) Int. Cl.: A61F 9/007

(54) **IMPLANTAT ZUM EINFÜHREN IN DEN SCHLEMMSCHEN KANAL EINES AUGES**
IMPLANT FOR INSERTING INTO THE SCHLEMM'S CANAL OF AN EYE
IMPLANT DESTINÉ À ÊTRE INSÉRÉ DANS LE CANAL DE SCHLEMM DE L'OEIL

(30) Priorität: 22.12.2008 CH 20052008; 12.11.2009 CH 17432009
(43) Veröffentlichungstag der Anmeldung: 31.08.2011
(73) Patentinhaber: Grieshaber Ophthalmic Research Foundation, 9000 St. Gallen (CH)
(72) Erfinder: STEGMANN, Robert, 0081 Pretoria (ZA); GRIESHABER, Matthias, 4102 Binningen (CH)
(74) Vertreter: Dr. Graf & Partner AG
(86) Internationale Anmeldenummer: PCT/EP2009/066814
(87) Internationale Veröffentlichungsnummer: WO 2010/072574

(56) Entgegenhaltungen:
- EP-A2- 0 898 947
- WO-A2-2004/026347
- US-A1- 2007 088 432

## Beschreibung

Die Erfindung bezieht sich auf ein Implantat zum Einführen in den durch einen Einschnitt und aufgeklappten Skleralappen freigelegten Schlemmschen Kanal eines Auges, bestehend aus einem länglichen bis mindestens ein viertel der Umfangsrichtung in das Lumen des zirkulären Schlemmschen Kanals biegsam einführbaren Röhrchen, welches mehrere im Abstand zueinander angeordnete und mit dem Innenraum in Verbindung stehende Öffnungen aufweist.

### Ophthalmologischer Hintergrund

Bei einem gesunden Auge erfolgt der Abfluss des zirkulierenden Kammerwassers (Humor aquosus) von der Hinterkammer zur Vorderkammer und wird im Kammerwinkel (Angulus iridocornealis) über das Trabekulargewebe in den Schlemmschen Kanal und von dort über das episklerale Venensystem in den Blutkreislauf abgeleitet. Bei krankhaften Zuständen des Auges, insbesondere bei auftretenden Widerständen aufgrund eines beispielsweise durch Verklebung oder dergleichen geschlossenen Schlemmschen Kanals, ist ein kontinuierlicher Abfluss des vom Epithel des Ziliarkörpers gebildeten und sich ständig erneuernden Kammerwassers oftmals gar nicht oder nicht mehr ausreichend gewährleistet. Hierdurch kann der Augeninnendruck (IOP) derart ansteigen, dass die Durchblutung der Sehnerven und somit die Funktion derselben eingeschränkt wird, wobei diese Funktionstörung zu der als Glaukom oder grüner Star bezeichneten Augenkrankheit und bis zum Erblinden des betroffenen Auges führen kann.

In Verbindung mit der allgemein bekannten Kanaloplastik-Methode besteht die Möglichkeit einer zirkumferentiellen Dilatation, bei welcher der Schlemmsche Kanal mittels eines eingeführten, flexiblen Mikrokatheters zirkulär gedehnt und gleichzeitig oder anschliessend mittels einer sogenannten Mikroschraube ein hochmolekulares Viskoclastikum injiziert wird. Anschliessend wird mit geeigneten Mitteln, beispielsweise mit einem chirurgischen Faden der zirkuläre Schlemmsche Kanal gegen die Vorderkammer hin gespannt und dadurch eine Dehnung des Trabekulargewebes sowie eine erhöhte Durchlässigkeit mit begünstigtem transtrabekulären Abfluss des Kammerwassers erreicht. Zur Optimierung des natürlichen transtrabekulären Kammerwasser-Abflusses kann anschliessend ein entsprechend ausgebildetes Implantat in den mechanisch gedehnten Schlemmschen Kanal eingeführt werden.

### Stand der Technik

Zum Verbessern und Aufrechterhalten des natürlichen transtrabekulären Kammerwasserabflusses sind aus der Druckschrift (EP 0 898 947 A2) unterschiedlich ausgebildete und in den freigelegten Schlemmschen Kanal biegsam einführbare längliche Stützelemente bekannt, welche beispielsweise als längliches Röhrchen mit mehreren am Umfang sowie in axialer Richtung in der Wand verteilt angeordneten Öffnungen, oder als Netzgeflecht, beziehungsweise als schraubenlinienförmiges Stützelement, oder als gitterförmig ausgebildetes Stützelement ausgebildet sind. Das im wesentlichen gitterförmig ausgebildete Stützelement hat zwei in axialer Richtung im Abstand zueinander angeordnete kreisringförmige Endstücke, welche mittels drei gleichmässig am Umfang verteilt daran befestigte und in axialer Richtung orientierte Stege derart miteinander verbunden sind, dass zwischen den einzelnen Stegen jeweils über die gesamte axiale Länge des Stützelementes orientierte Durchgänge vorgesehen sind.

Weiterhin sind zur Aufrechterhaltung des natürlichen transtrabekulären Kammerwasserabflusses aus der Druckschrift (EP 1 125 568 A2) unterschiedlich ausgebildete und in den freigelegten Schlemmschen Kanal einführ- und implantierbare Stützelemente bekannt, welche beispielsweise ringförmig, kugelförmig oder röhrchenförmig ausgebildet und aus einem von dem Gewebe des Schlemmschen Kanals oder von dem Kammerwasser abbaubarem Material hergestellt sind.

Zur Reduzierung des Augen-Innendrucks (IOP) sind aus den US-Druckschriften (2004/0210181 A1; 2007/088432 A1) als Bypass oder als Dränage im wesentlichen T-förmig ausgebildete Abzweigelemente bekannt, welche mit einem proximalen Röhrchen durch das Trabekulargewebe direkt in die Vorderkammer des Auges und mit zwei am distalen Ende angeordneten und mit Öffnungen versehenen distalen Röhrchen in den Schlemmschen Kanal derart eingeführt werden, dass das Kammerwasser von der Vorderkammer in das proximale Röhrchcn und von den beiden distalen Röhrchen in den Schlemmschen Kanal sowie über das episklerale Venensystem in den Blutkreislauf des Auges abgeleitet wird. Mit diesem als Bypass oder Dränage ausgebildeten Abzweigelement kann das Kammerwasser auf künstlichem Weg direkt von der Vorderkammer in den Schlemmschen Kanal und anschliessend über das episklerale Venensystem in den Blutkreislauf abgeleitet werden.

### Darstellung der Erfindung

Der Erfindung liegt die Aufgabe zugrunde ein in den Schlemmschen Kanal einführbares Implantat der eingangs genannten Art dahingehend auszubilden und zu verbessern, dass ohne zusätzliche Hilfsmittel, beispielsweise in Form einer medikamentösen Therapie, eine den Augeninnendruck auf einen konstanten Wert regulierende Zirkulation des Kammerwassers über das gesamte Lumen des zirkulären Schlemmschen Kanals erreicht und der anatomisch natürliche transtrabekuläre Abfluss des sich ständig erneuernden Kammerwassers über das episklerale Venensystem in den Blutkreislauf des Auges verbessert und permanent aufrechterhalten wird.

Die Aufgabe wird gelöst mit einem Implantat umfassend die Merkmale von Anspruch 1.

Ausführungsbeispiele und Merkmale sowie Einzelheiten der Erfindung ergeben sich aus der nachstehenden Beschreibung in Verbindung mit der Zeichnung und den einzelnen Patentansprüchen.

Das erfindungsgemässe und in das Lumen des Schlemmschen Kanals eingeführte Implantat hat den Vorteil, dass der Schlemmsche Kanal permanent offen gehalten und stabilisiert wird. Das Implantat erstreckt sind mindestens in halber Umfangsrichtung vorzugsweise aber in gesamter Umfangsrichtung des zirkulären Schlemmschen Kanals, so dass dieser über den gesamten Umfang offen gehalten wird und der anatomisch natürliche transtrabekulare Abfluss des Kammerwassers über das episklerale Venensystem in den Blutkreislauf hergestellt und die Regulierung des Augeninnendrucks (IOP) gewährleistet ist.

Zur Optimierung des transtrabekulären Kammerwasserabflusses besteht die Möglichkeit, dass in einer ersten Phase der Schlemmsche Kanal durch eine zirkumferentielle Dilatation gedehnt und anschliessend das erfindungsgemässe Implantat in das gedehnte Lumen eingeführt und am Zielort freigesetzt wird.

### Beschreibung der Zeichnungen

Die zur Erläuterung der Ausführungsbeispiele verwendeten Zeichnungen zeigen:
- Fig. 1: einen schematisch und in grösserem Massstab dargestellten Längsschnitt durch ein Auge;
- Fig. 2: eine schematisch dargestellte Frontansicht des Auges mit einem parabolischen Einschnitt in der Sklera und aufgeklapptem Skleralappen;
- Fig. 3: das gemäss der in Fig.2 eingezeichneten Schnittlinie A-A in grösserem Massstab dargestellte Teilstück des Auges mit teilweise freigelegtem Schlemmschen Kanal;
- Fig. 4: ein in grösserem Massstab dargestelltes Teilstück des Auges mit in den Schlemmschen Kanal eingeführter Injektionssonde;
- Fig. 5: einen in grösserem Massstab dargestellten Teilabschnitt des freigelegten Schlemmschen Kanals gemäss Fig.4 mit einem in das Lumen desselben eingeführten und freigesetzten Implantat;
- Fig. 6: ein erstes in Ansicht dargestelltes Ausführungsbeispiel des aus einem kreisring- oder ovalringförmigen Röhrchen hergestellten Implantats;
- Fig. 7: das gemäss der in Fig.6 eingezeichneten Schnittlinie B-B in grösserem Massstab im Querschnitt dargestellte und kreisringförmig ausgebildete Implantat;
- Fig. 8: eine in grösserem Massstab dargestellte Variante des im Querschnitt ovalringförmig ausgebildeten Implantats gemäss der Schnittlinie B-B in Fig.6;
- Fig. 9: ein in Ansicht dargestelltes zweites Ausführungsbeispiel des aus einem kreisring- oder ovalringförmigen länglichen Röhrchen hergestellten Implantats;
- Fig. 10: das in grösserem Massstab dargestellte und im Querschnitt kreisringförmig ausgebildete Implantat gemäss der Schnittlinie C-C in Fig.9;
- Fig. 11: eine Variante des im Querschnitt kreisringförmig ausgebildeten Implantats gemäss Figur 10;
- Fig. 12: eine in grösserem Massstab dargestellte Variante des im Querschnitt ovalringförmig ausgebildeten Implantats gemäss der Schnittlinie C-C in Fig.9;
- Fig. 13-16: weitere Ausführungsbeispiele des ovalringförmig ausgebildeten Implantats gemäss der Schnittlinie C-C in Fig.9; und
- Fig. 17-18: weitere Varianten des in Fig.6 dargestellten Implantats.

### Figurenbeschreibung

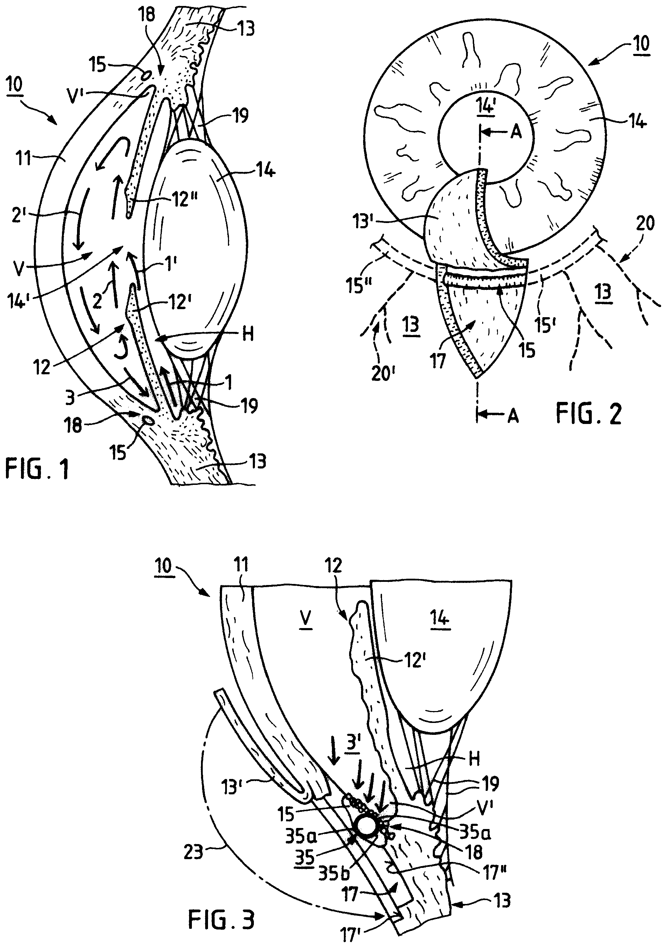

An dieser Stelle wird darauf hingewiesen, dass in den Figuren 1 bis 5 jeweils ein Teilstück des Auges zum besseren Verständnis der Problematik in Verbindung mit der Glaukomchirurgie dargestellt ist. Weiterhin sind in den einzelnen Figuren sowie in der nachstehenden Beschreibung gleiche Teile mit gleichen Bezugszeichen versehen.

Fig.1 zeigt in der bereits aus der Druckschrift EP 0 898 947 bekannten Darstellung einen schematisch dargestellten Vertikalschnitt des vorderen Abschnittes eines Auges 10 und man erkennt die Hornhaut 11 (Cornea), die Regenbogenhaut 12 (Iris) mit den beiden Bereichen 12' und 12", die Lederhaut 13 (Sklera), die Linse 14 mit der Pupille 14', die Zonulafasern 19, den zirkulären Schlemmschen Kanal 15 (Sinus venosus sclerae) sowie das dem Schlemmschen Kanal 15 vorgelagerte Trabekulargewebe 18 (Trabeculum corneosclerale).

Wie in Fig.1 schematisch dargestellt, erfolgt bei einem gesunden Auge der Abfluss des zirkulierenden und sich ständig erneuernden Kammerwassers (Humor aquosus) gemäss der eingezeichneten Pfeile 1,1' und 2,2" von der Hinterkammer H zu der Vorderkammer V und wird im Kammerwinkel V' (Angulus iridocornealis) gemäss Pfeilrichtung 3 über das Trabekulargewebe 18 in das Lumen des zirkulären Schlemmschen Kanals 15 abgeleitet und gelangt von dort über das in Fig.1 nicht dargestellte episklerale Venensystem wieder in den Blutkreislauf.

Wie eingangs erwähnt ist bei krankhaften Zuständen des betroffenen Auges ein kontinuierlicher Abfluss des vom Epithel des Ziliarkörpers gebildeten und sich ständig erneuernden Kammerwassers oftmals nicht mehr gewährleistet. Der Schlemmsche Kanal 15 kann sich derart verschliessen, dass der Abfluss des Kammerwassers behindert beziehungsweise weitgehend ausgeschlossen ist, so dass der Augeninnendruck derart ansteigt, dass die Durchblutung der Sehnerven und in Folge davon die Funktion derselben bis zum Erblinden des betroffenen Auges eingeschränkt wird.

Figur 2 zeigt eine ebenfalls aus der Druckschrift EP 0 898 947 an sich bekannte Darstellung des Auges 10 in schematischer Frontansicht und man erkennt die Linse 14 mit der Pupille 14', ein Teilstück der Lederhaut 13, einen Teilabschnitt des Schlemmschen Kanals 15 sowie ein Teilstück des damit in Verbindung stehenden natürlichen Kanalsystems 20,20' (Kammerwasser-Kanalsystem). Der in Fig.2 teilweise und schematisch dargestellte Schlemmsche Kanal 15 erstreckt sich in Umfangsrichtung über einen Winkel von 360° und verläuft zirkulär um die Linse 14.

Mit einem mikrochirurgischen Eingriff wird in an sich bekannter Weise, wie in Fig.2 schematisch dargestellt, die Lederhaut 13 lamellar eingeschnitten und nach dem Abtrennen eines nicht näher dargestellten Lederhaut-Teilstücks das äussere lappenförmige Teilstück 13' aufgeklappt und für den weiteren operativen Eingriff mit nicht dargestellten Mitteln gehalten. Der lamellare Einschnitt bildet im Bereich des freigelegten Schlemmschen Kanals 15 ein mit 17 bezeichnetes Sklerabett, welches nach erfolgtem Eingriff, beispielsweise nach dem Einführen und Freisetzen eines länglichen Implantats, mit dem gemäss Pfeilrichtung 23 (Fig.3) herunterklappbaren Teilstück 13'(Skleralappen) wieder geschlossen wird.

Bei einer weiteren Variante des mikrochirurgischen Eingriffs besteht jedoch auch die Möglichkeit, dass das dem zirkulären Schlemmschen Kanal 15 vorgelagerte Trabekulargewebe 18 (Fig.3) zum Einführen und Freisetzen des Implantats mit einem in nicht dargestellter Weise in die Vorderkammer V eingeführten Schneidinstrument oder dergleichen mindestens teilweise zirkulär geöffnet wird.

Figur 3 zeigt das gemäss der in Fig.2 eingezeichneten Linie A-A im Schnitt sowie in grösserem Massstab dargestellte Teilstück des Auges 10 und man erkennt die Hornhaut 11, den ersten Bereich 12' der Regenbogenhaut 12, die Lederhaut 13 mit dem aufgeklappten Skleralappen 13', die Linse 14, die Zonulafasern 19, die Hinterkammer H und die Vorderkammer V mit dem Kammerwinkel V', das Trabekulargewebe 18 und den Schlemmschen Kanal 15 mit dem darin angeordneten Implantat 35. Der zirkulär um die Linse 14 orientierte Schlemmsche Kanal 15 erstreckt sich, wie in Fig.3 schematisch und in grösserem Massstab dargestellt, im wesentlichen entlang des Trabekulargewebes 18 und ist im Profilquerschnitt etwa als längliches Oval ausgebildet, welches ausgehend von dem einen Ende im Bereich des Kammerwinkels V' in Richtung des anderen Endes eine im wesentlichen sich verjüngende Formgebung hat. Weiterhin erkennt man in Fig.3 das durch den Einschnitt freigelegte Sklerabett 17 mit der Innenfläche 17" und der Auflagefläche 17" für den Skleralappen 13'.

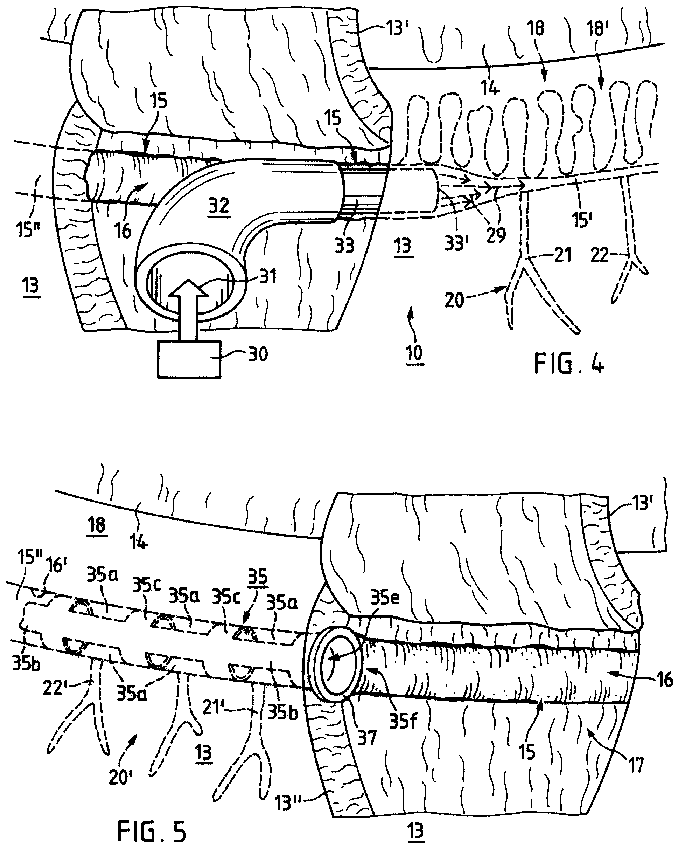

In Fig.4 ist in an sich bekannter Weise zur Dehnung des Schlemmschen Kanals 15 eine röhrchenförmige und an einem Anschlussstück 32 angeordnete Sonde 33 in das Lumen 16 des freigelegten Schlemmschen Kanals 15 eingeführt. Das Anschlussstück 32 steht über eine nicht dargestellte Zuführleitung mit einem schematisch dargestellten Injektionsgerät 30 in Verbindung. Mit dem Injektionsgerät 30 wird über die am distalen Ende mit mindestens einer Austrittsöffnung 33' versehene röhrchenförmige Sonde 33 beispielsweise eine hydrophile Flüssigkeit 29 gemäss Pfeilrichtung 31 in den Schlemmschen Kanal 15 injiziert und infolge davon ein in Umfangsrichtung orientiertes Teilstück 15' des Schlemmschen Kanals 15 hydraulisch gedehnt.

Weiterhin kann in an sich bekannter Weise mit einer spiegelbildlich ausgebildeten und in den Schlemmschen Kanal 15 eingeführten Sonde das dem bereits behandelten Teilstück 15' gegenüberliegende Teilstück 15" des Schlemmschen Kanals 15 analog behandelt und in zirkulärer Richtung hydraulisch gedehnt werden. Weiterhin erkennt man in Fig.4 das dem Schlemmschen Kanal 15 vorgelagerte Trabekulargewebe 18 (trabecular meshwork) mit den schematisch dargestellten Gewebebälkchen 18' sowie das Kanalsystem 20 mit den Kanälchen 21 und 22.

Bei der vorstehend beschriebenen Dehnung des Schlemmschen Kanals 15 werden gegebenenfalls in der Wandung entstehende Öffnungen (nicht dargestellt) gleichzeitig mit der hydrophilen Flüssigkeit 29 beschichtet, so dass die an den Wandungen der Öffnungen in Form eines Films haftende hydrophile Flüssigkeit eine lokale Gewebeverbindung verhindert und den Abfluss des Kammerwassers gewährleistet. Anstelle der hydrophilen Flüssigkeit kann auch ein geeignetes, biologisch verträgliches gasförmiges Medium oder aber ein Gemisch aus der hydrophilen Flüssigkeit und dem gasförmigen Medium zum Dehnen des Schlemmschen Kanals verwendet werden.

Wie in Fig.5 schematisch dargestellt wird im Anschluss an die hydraulische oder pneumatische Dehnung und zur Optimierung einer dauerhaften Durchlässigkeit und Zirkulation des Kammerwassers ein Implantat 35 in das Lumen 16 des zirkulären Schlemmschen Kanals 15 eingeführt. Das Implantat 35 besteht aus einem länglichen biegsamen Röhrchen 36 und ist vorzugsweise aus biokompatiblem, elastischem Material hergestellt und wird mit entsprechenden nicht näher dargestellten Mitteln, beispielsweise mittels einer Sonde (inserting instrument) oder dergleichen in das Lumen 16 des Schlemmschen Kanals 15 eingeführt.

Fig.5 zeigt weiterhin ein Teilstück des in den Schlemmschen Kanal 15 eingeführten Implantats 35, welches mit dem nicht dargestellten proximalen Ende (nearest to the inserting instrument) lösbar an der Sonde (inserting instrument) angeordnet ist. An dem anderen, distalen Ende (farthest from the inserting instrument) ist das Implantat 35 mit einem an der Innenseite 13" der Lederhaut 13 anliegenden und eine Öffnung 35f aufweisenden Anlagebund 37 versehen. Das in das Lumen 16 eingeführte Implantat 35 erstreckt sich von der einen Innenseite 13" des freigelegten Schlemmschen Kanals 15 in nicht näher dargestellter Weise bis mindestens in viertel, halber, dreiviertel oder vorzugsweise in ganzer Umfangsrichtung bis auf die gegenüberliegende Innenseite des lamellaren Einschnitts (Fig.2). Bei einer nicht dargestellten Variante besteht weiterhin die Möglichkeit, von der einen Seite und von der anderen gegenüberliegenden Seite des lamellaren Einschnitts jeweils ein im wesentlichen halbkreisförmig gebogenes Implantat 35 in den freigelegten zirkulären Schlemmschen Kanal 15 einzuführen. Mit dem Implantat 35 wird das Lumen 16 des zirkulären Schlemmschen Kanals 15 gestützt und permanent offengehalten.

In Fig.5 ist weiterhin das durch den lamellaren Einschnitt zwischen den beiden gegenüberliegenden Innenseiten 13" gebildete Sklerabett 17 dargestellt, welches bei heruntergeklapptem sowie auf die parabolförmige Auflagefläche 17" gelegten und entsprechend mit der Lederhaut 13 vernähten Skleralappen 13' einen subskleralen Raum beziehungsweise ein Sammelbecken (Reservoir) für das Kammerwasser bildet. Das Sklerabett 17 steht über die beiden gegenüberliegend zueinander angeordneten Öffnungen 35f (nur eine Öffnung 35f dargestellt) des Implantats 35 mit dem Innenraum 35e desselben in Verbindung.

Weiterhin erkennt man in Fig.5 ein Teilstück des in den Schlemmschen Kanal 15 eingeführten Implantats 35, welches mit im Abstand zueinander angeordneten Ringteilen 35c abstützend an der Innenwand 16' des Lumens 16 anliegt. Zwischen den einzelnen Ringteilen 35c angeordnete Öffnungen oder Ausnehmungen 35a bilden, wie in Fig.5 dargestellt, jeweils eine direkte und permanent offengehaltene Verbindung zwischen dem Trabekulargewebe 18 und den einzelnen Kanälchen 21' und 22' des Kanalsystems 20', so dass der natürliche transtrabekuläre Abfluss des Kammerwassers von der Vorderkammer V über das Trabekulargewebe 18 in den zirkulären Schlemmschen Kanal 15 beziehungsweise in den Innenraum 35e des Implantats 35 und von dort über das episklerale Venensystem in den Blutkreislauf gewährleistet ist.

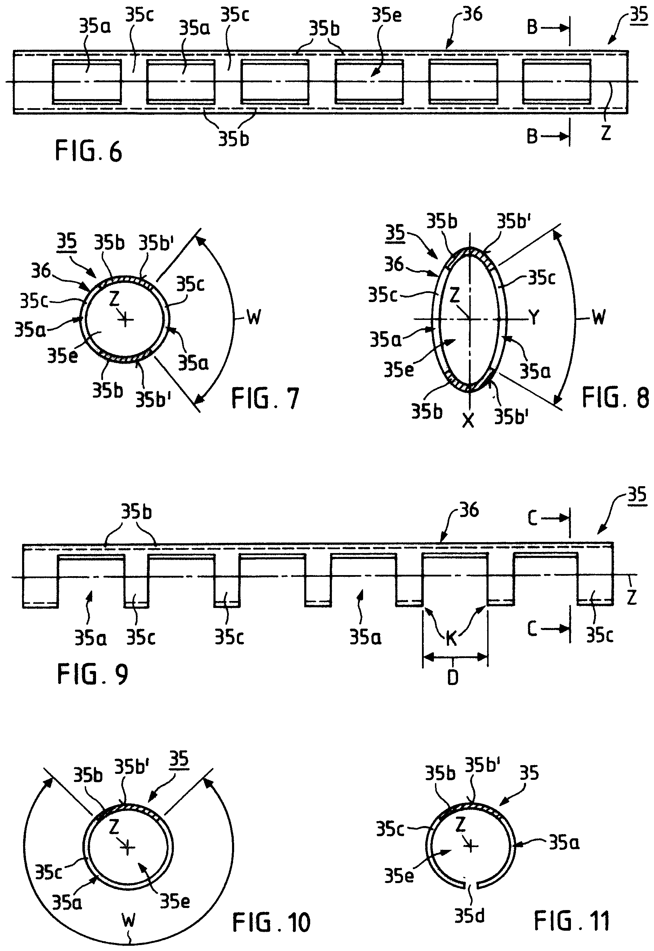

Figur 6 zeigt ein erstes Ausführungsbeispiel des aus einem biegsamen Röhrchen 36 hergestellten Implantats 35, welches zwei am Umfang gegenüberliegend zueinander angeordnete und durchgehend in Richtung der Längsachse Z orientierte Verbindungsteile 35b sowie in Richtung der Längsachse Z im Abstand zueinander angeordnete Ringteile 35c aufweist. Zwischen den einzelnen Ringteilen 35c ist jeweils eine mit dem Innenraum 35e des Implantats 35 in Verbindung stehende Öffnung 35a angeordnet. In dem dargestellten Ausführungsbeispiel sind die mit dem Innenraum 35e in Ver-bindung stehenden Öffnungen 35a viereckig ausgebildet, wobei die Öffnungen 35a jedoch auch beliebig, beispielsweise oval, elliptisch, quadratisch oder trapezförmig ausgebildet werden können.

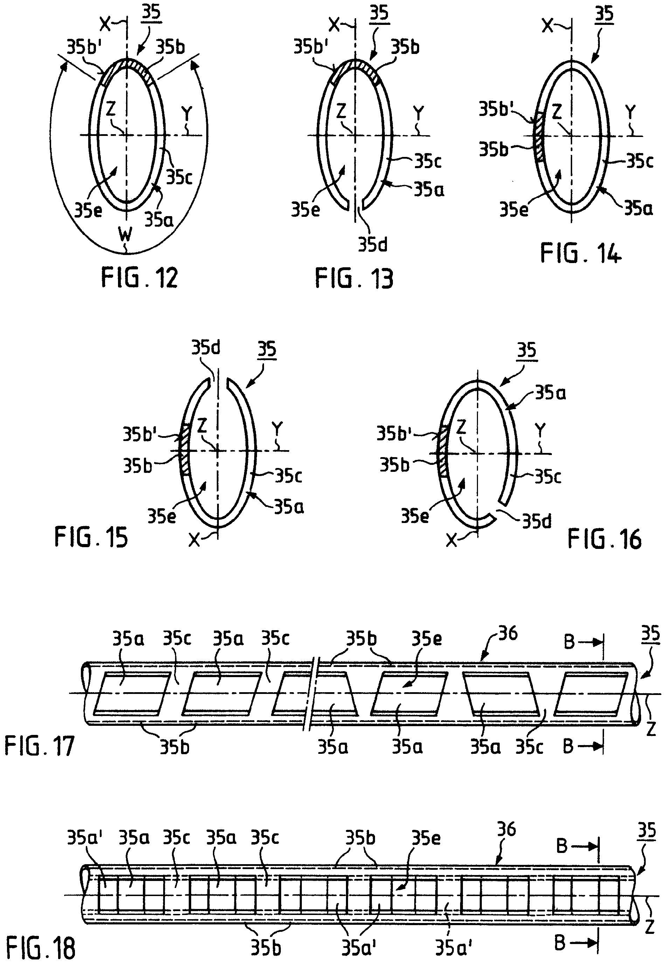

An dieser Stelle wird darauf hingewiesen, dass die Öffnungen 35a sowie die dazwischen angeordneten und stegförmig ausgebildeten Ringteile 35c, wie in Fig.17 dargestellt, in Bezug auf die Längsachse Z entweder in die eine oder in die andere Richtung geneigt ausgebildet werden können, wobei die einzelnen Ringteile 35c parallel im Abstand zueinander angeordnet sind. Weiterhin besteht die Möglichkeit, dass die Ringteile 35c mit den dazwischen angeordneten Öffnungen 35a in Bezug auf die Längsachse Z abwechselnd gegenläufig geneigt zueinander angeordnet sind. Wie in Fig.18 dargestellt können die an der einen Seite des Röhrchens 36 in Reihe angeordneten Öffnungen 35a sowie die an der anderen gegenüberliegenden Seite des Röhrchens 36 in Reihe angeordneten Öffnungen 35a' auch in Richtung der Längsachse Z versetzt zueinander angeordnet werden.

Die stegförmigen Ringteile 35c sind vorzugsweise schmal und die in axialer Richtung orientierten Öffnungen 35a oder Ausnehmungen 35a relativ gross ausgebildet, so dass bei eingeführtem Implantat 35, wie vorstehend erwähnt, das Trabekulargewebe 18 sowie die einzelnen Kanälchen 21', 22' des Kanalsystems 20" frei liegen und somit der natürliche transtrabekuläre Abfluss des Kammerwassers gewährleist ist (Fig.5).

Figur 7 zeigt das in grösserem Massstab und im Profilquerschnitt kreisringförmig ausgebildete Implantat 35 gemäss der in Figur 6 eingezeichneten Schnittlinie B-B und man erkennt die beiden am Umfang gegenüberliegend zueinander angeordneten und in Richtung der Längsachse Z orientierten Verbindungsteile 35b. Weiterhin erkennt man die jeweils zwischen den beiden Verbindungsteilen 35b in Umfangsrichtung angeordneten und mit dem Innenraum 35e in Verbindung stehenden Öffnungen 35a. Die gegenüberliegend zueinander zwischen den einzelnen stegförmigen Ringteilen 35c angeordneten Öffnungen 35a haben bei diesem Ausführungsbeispiel jeweils einen zwischen 90° und 105° begrenzten Öffnungswinkel W. Die Verbindungsteile 35b sind mit einer in Richtung der Längsachse Z orientierten kreisbogenförmigen Oberfläche 35b' versehen, welche zur Anlage an der Innenwand 16' des Lumens 16 ausgebildet ist (Fig.5).

Fig. 8 zeigt eine in grösserem Massstab dargestellte Variante des Implantats 35 gemäss der in Fig.6 eingezeichneten Schnittlinie B-B. Abweichend von dem in Fig.7 dargestellten Ausführungsbeispiel ist dieses Implantat 35 im Profilquerschnitt ovalringförmig, vorzugsweise als doppelt symmetrisches ringförmiges Oval ausgebildet, welches zwei orthogonal zu der Längsachse Z orientierte Symmetrieachsen X und Y aufweist. Das als doppelt symmetrisches und ringförmiges Oval ausgebildete Implantat 35 hat zwei an den kleineren kreisbogenförmigen Enden gegenüberliegende und in Richtung der Längsachse Z orientierte Verbindungsteile 35b mit bogenförmig ausgebildeter Oberfläche 35b'. Weiterhin erkennt man in Fig.8 die an den grösseren kreisbogenförmigen Seiten gegenüberliegend zueinander angeordneten und mit dem Innenraum 35e in Verbindung stehenden Öffnungen 35a, welche im dargestellten Ausführungsbeispiel jeweils einen zwischen 90° und 105° begrenzten Öffnungswinkel W aufweisen.

Figur 9 zeigt ein weiteres Ausführungsbeispiel des aus dem biegsamen Röhrchen 36 hergestellten Implantats 35, welches abweichend von dem in Figur 6 dargestellten Ausführungsbeispiel nur ein einziges durchgehend in Richtung der Längsachse Z orientiertes Verbindungsteil 35b sowie mehrere beabstandete Ringteile 35c und dazwischen angeordnete Öffnungen 35a aufweist. Bei diesem Ausführungsbeispiel sind die Öffnungen 35a jeweils als eine von der einen Seite bis zu der anderen Seite des Verbindungsteils 35b reichende Ausnehmung 35a ausgebildet. Der zwischen den einzelnen stegförmigen Ringteilen 35c vorgesehene Abstand D ist so gewählt, dass in kreisförmig gebogenem Zustand (nicht dargestellt) des Implantats 35 die einander zugewandten Kanten K der stegförmigen Ringteile 35c noch im Abstand zu einander angeordnet sind. Hierdurch wird erreicht, dass das in den Schlemmschen Kanal 15 eingeführte Implantat 35 eine in Umfangsrichtung orientierte gleichmässige Lage aufweist und ein Verkanten der einzelnen Ringteile 35c ausgeschlossen ist. Die einzelnen in axialer Richtung der Längsachse Z im Abstand zueinander angeordneten Ringteile 35c sind an der Aussenseite analog dem Verbindungsteil 35b jeweils mit einer bogenförmigen Oberfläche 35b' versehen. Die in Fig.9 in parallelem Abstand zueinander angeordneten Ringteile 35c können in axialer Richtung entweder in die eine oder in die andere Richtung geneigt oder aber abwechselnd gegenläufig geneigt zueinander ausgebildet werden.

Figur 10 zeigt das gemäss der Schnittlinie C-C in Fig.9 im Profilquerschnitt kreisringförmig ausgebildete Implantat 35 und man erkennt das in Richtung der Längsachse Z orientierte und mit einer bogenförmigen Oberfläche 35b' versehene Verbindungsteil 35b sowie die mit einem Öffnungswinkel W von 280° bis 290° versehene und mit dem Innenraum 35e in Verbindung stehende Ausnehmung 35a.

In Fig.11 ist eine Variante des Implantats 35 gemäss der in Fig.9 eingezeichneten Schnittlinie C-C im Profilquerschnitt dargestellt, bei welchem die einzelnen Ringteile 35c des kreisringförmig ausgebildeten Implantats 35 an der dem Verbindungsteil 35b gegenüberliegenden Seite jeweils durch einen in axialer Richtung orientierten Spalt 35d durchtrennt sind. Die einzelnen Ringteile 35c können, beispielsweise zum lösbaren Verbinden mit einer nicht dargestellten Sonde (inserting instrument) oder dergleichen relativ zueinander aufgebogen und durch die eigene federelastische Rückstellkraft wieder in die ursprünliche Lage zurückgeführt werden.

Eine weitere Variante des Implantats 35 gemäss der in Fig.9 eingezeichneten Schnittlinie C-C ist in Fig. 12 in grösserem Massstab dargestellt. Abweichend von dem in Fig.10 oder 11 dargestellten Ausführungsbeispiel ist das Implantat 35 gemäss Fig.12 im Profilquerschnitt ovalringförmig, vorzugsweise als doppelt symmetrisches ringförmiges Oval ausgebildet, welches die Längsachse Z sowie die beiden im wesentlichen orthogonal dazu angeordneten Symmetrieachsen X und Y aufweist. Bei dieser Variante ist das mit der bogenförmigen Oberfläche 35b' versehene und in Richtung der Längsachse Z orientierte Verbindungsteil 35b an dem oberen bogenförmig ausgebildeten Teilstück des Ovals angeordnet. Bei einer nicht näher dargestellten Variante kann das Verbindungsteil 35b jedoch auch an dem gegenüberliegenden unteren bogenförmig ausgebildeten Teilstück des Ovals angeordnet werden.

Fig.13 zeigt eine Variante des Implantats 35 gemäss Fig.12, bei welchem die in Richtung der Längsachse Z im Abstand zueinander angeordneten Ringteile 35c an dem einen dem Verbindungsteil 35b gegenüberliegenden Ende des Ovals jeweils durch einen Spalt 35d getrennt sind. Die einzelnen Ringteile 35c können somit in nicht näher dargestellter Weise relativ zueinander aufgebogen und durch die eigene federelastische Rückstellkraft wieder in die ursprüngliche Lage zurückgeführt werden.

In Fig. 14 ist ein weiteres Ausführungsbeispiel des im Profilquerschnitt ovalringförmig, vorzugsweise als doppelt symmetrisches ringförmiges Oval ausgebildetes Implantat 35 dargestellt, welches die Längsachse Z sowie die beiden Symmetrieachsen X und Y aufweist. Bei diesem Implantat 35 ist das mit der bogenförmigen Oberfläche 35b' versehene und in Richtung der Längsachse Z orientierte Verbindungsteil 35b an der einen oder an der gegenüberliegenden anderen bogenförmigen Seite angeordnet.

Fig.15 zeigt eine Variante des Implantats 35 gemäss Fig.14, bei welchem die im Abstand zueinander an dem in Richtung der Längsachse Z orientierten Verbindungsteil 35b angeordneten Ringteile 35c an dem einen kleineren bogenförmigen Ende des Ovals jeweils durch einen Spalt 35d getrennt sind. Der Spalt 35d kann jedoch auch an dem anderen gegenüberliegenden bogenförmigen Ende des Ovals angeordnet werden.

Fig.16 zeigt ein weiteres Ausführungsbeispiel des Implantats 35, bei welchem abweichend von der in Fig.15 dargestellten Variante die im Abstand zueinander an dem in Richtung der Längsachse Z orientierten Verbindungsteil 35b angeordneten Ringteile 35c jeweils durch einen in Umfangsrichtung des Ovals an beliebiger Stelle angeordneten Spalt 35d getrennt sind.

Bei dem jeweils in den Figuren 13, 15 und 16 dargestellten Implantat 35 besteht die Möglichkeit, dass die einzelnen durch den Spalt 35d getrennten Ringteile 35c, beispielsweise zum lösbaren Verbinden mit einer nicht dargestellten Sonde (inserting instrument), in Bezug auf die Symmetrieachse X relativ zueinander aufgebogen werden können und durch die eigene federelastische Rückstellkraft wieder in die ursprüngliche Lage zurückführbar sind.

Bei dem in den Figuren 12 bis 16 dargestellten und jeweils als doppelt symmetrisches ringförmiges Oval ausgebildeten Implantat 35 sind die zwischen den stegförmigen Ringteilen 35c angeordneten Ausnehmungen 35a jeweils mit einem in Umfangsrichtung des Ovals orientierten Öffnungswinkel W von 280° bis 290° versehen.

Das jeweils vorstehend in Verbindung mit den einzelnen Figuren 6 bis 18 beschriebene und aus einem länglichen Röhrchen hergestellte Implantat 35 besteht aus biologisch verträglichem Material, wie Gold, Nitinol oder dergleichen beziehungsweise aus biologisch verträglichem flexiblem Material, beispielsweise aus polymerem Material mit thermischem oder mechanischem Formgedächtniseffekt. Hiermit wird erreicht, dass das beispielsweise etwa dem Schlemmschen Kanal 15 kreisringförmig ausgebildete Implantat 35 vor dem Einführen bei einer Raumtemperatur von etwa 18°C bis 22°C weitgehend geradlinig aufgebogen in den Schlemmschen Kanal 15 eingeführt werden kann und infolge der Körpertemperatur von etwa 35°C bis 37°C in die an der Innenwand 16' des Schlemmschen Kanals 15 entsprechend abstützend anliegende Form zurückführbar ist. Weiterhin besteht die Möglichkeit, dass das Implantat 35 vor dem Einführen oder während des Einführens in den Schlemmschen Kanal 15 bei einer Raumtemperatur von etwa 18°C bis 22°C in nicht näher dargestellter Weise geringfügig quer zur Längsachse Z zusammengedrückt und infolge der Körpertemperatur von etwa 35°C bis 37°C in die ursprünglich kreisring- oder ovalringförmige Form zurückführbar ist.

Weiterhin besteht die Möglichkeit, dass das aus flexiblem Material hergestellte Implantat 35 zwei in axialer Richtung gegenüberliegend zueinander angeordnete und in axialer in Richtung orientierte Verbindungsteile 35b (Fig.7,8) aufweist, oder mit nur einem in axialer Richtung orientierten Verbindungsteil 35b (Fig.10 bis 16) versehen ist. Bei einer bevorzugten Ausführungsform besteht die Möglichkeit, dass das aus dem länglichen Röhrchen 36 hergestellte und im Profilquerschnitt kreisringförmig oder ovalringförmig ausgebildete Implantat 35 mit einer Heparin-Beschichtung versehen ist.

Zum Einführen des Implantats 35 in das Lumen 16 des Schlemmschen Kanals 15 wird dieser zunächst entsprechend der an sich bekannten und eingangs erwähnten Kanaloplastik-Methode mittels eines flexiblen Mikrokatheters schonend zirkulär gedehnt und gleichzeitig oder anschliessend mittels einer sogenannten Mikroschraube ein hochmolekulares Viskoelastikum injiziert. Nach erfolgter Dilatation wird der Mikrokatheter wieder zurückgezogen und gleichzeitig der Schlemmsche Kanal 15 etwa in Richtung der Vorderkammer V hin gespannt und dadurch eine Dehnung des Trabekulargewebes 18 erreicht, so dass das Implantat 35 eingeführt werden kann. Mit dem erfindungsgemässen und vorstehend im Einzelnen beschriebenen sowie in den einzelnen Figuren 6 bis 18 dargestellten und etwa als kreisförmiger Ring biegsamen Implantat 35 (Kanaloplastik-Ring) wird einerseits eine permanente Dehnung des Trabekelwerkes erreicht und andererseits das Lumen 16 des zirkulären Schlemmschen Kanals 15 in Umfangsrichtung für den transtrabekulären Abfluss des Kammerwassers permanent offen gehalten und stabilisiert.

## Patentansprüche

1. Implantat zum Einführen in den Schlemmschen Kanal (15) eines Auges (10), bestehend aus einem sich in Richtung einer Längsachse (Z) erstreckenden, biegsamen Röhrchen (36), wobei das Röhrchen (36) bis mindestens ein Viertel der Umfangsrichtung des Lumens (16) des zirkulären Schlemmschen Kanals (15) einführbar ist, wobei das Röhrchen (36) mehrere Öffnungen (35a) aufweist, welche in Richtung der Längsachse (Z) im Abstand zueinander angeordneten sind, **dadurch gekennzeichnet, dass** das Röhrchen (36) am Umfang ein in axialer Richtung verlaufendes Verbindungsteil (35b) oder zwei in axialer Richtung verlaufende, bezüglich der Längsachse (Z) diametral zueinander angeordnete Verbindungsteile (35b) umfasst, dass das Röhrchen (36) mehrere in axialer Richtung im Abstand zueinander angeordnete, vom Verbindungsteil (35b) ausgehende, stegförmige Ringteile (35c) umfasst, wobei die Öffnungen (35a) zwischen den einzelnen Ringteilen (35c) angeordnet sind, und wobei die Öffnungen (35a) eine Verbindung mit dem Innenraum (35e) des Röhrchens (36) ausbilden, wobei jede Öffnung (35a) in Umfangsrichtung des Röhrchens (36) zwischen dem Verbindungsteil (35b) angeordnet ist und jeweils einem in Umfangsrichtung orientierten Öffnungswinkel (W) aufweist.

2. Implantat nach Anspruch 1, **dadurch gekennzeichnet, dass** das Röhrchen (36) zwei in axialer Richtung verlaufende Verbindungsteile (35b) umfasst, ein erstes und ein zweites Verbindungsteil (35b) wobei das erste und das zweite Verbindungsteil (35b) bezüglich der Längsachse (Z) gegenüberliegend angeordnet sind.

3. Implantat nach Anspruch 2, **dadurch gekennzeichnet, dass** die zwischen dem ersten und dem zweiten Verbindungsteil (35b) sowie zwischen den einzelnen im Abstand zueinander angeordneten Ringteilen (35c) am Umfang des Röhrchens (36) vorgesehenen Öffnungen (35a) jeweils mit einem Öffnungswinkel (W) in der Grössenordnung zwischen 90° bis 105° versehen sind.

4. Implantat nach einem der vorhergehenden Ansprüche, **dadurch gekennzeichnet, dass** die zwischen dem Verbindungsteil (35b) verlaufenden, in Richtung der Längsachse (Z) gegenseitig beabstandet zueinander angeordneten Ringteilen (35c) senkrecht zur Längsachse (Z) verlaufen, und zwischen den Ringteilen (35c) am Umfang des Röhrchens (36) Öffnungen mit jeweils in Umfangsrichtung orientierten Ausnehmungen (35a) ausbilden.

5. Implantat nach Anspruch 1, **dadurch gekennzeichnet, dass** die am Umfang des Röhrchens (36) angeordneten Ausnehmungen (35a) jeweils mit einem Öffnungswinkel (W) in der Grössenordnung zwischen 280° bis 290° versehen sind.

6. Implantat nach Anspruch 1, **dadurch gekennzeichnet, dass** die in axialer Richtung im Abstand zueinander an dem ersten Verbindungsteil (35b) angeordneten stegförmigen Ringteile (35c) jeweils mit einem schlitzförmigen Spalt (35d) versehen ist und dadurch zwei Teilstücke ausbilden, wobei die beiden Teilstücke relativ zueinander spreizbar sind.

7. Implantat nach einem der Ansprüche 1 bis 6, **dadurch gekennzeichnet, dass** das mit den parallel zueinander angeordneten Verbindungsteilen (35b), den stegförmigen Ringteilen (35c) und den dazwischen angeordneten Öffnungen oder Ausnehmungen (35a) versehene längliche Röhrchen (36) im Profilquerschnitt kreisringförmig ausgebildet ist.

8. Implantat nach einem der Ansprüche 1 bis 6, **dadurch gekennzeichnet, dass** das mit den parallel zueinander angeordneten Verbindungsteilen (35b), den stegförmigen Ringteilen (35c) und den dazwischen angeordneten Öffnungen oder Ausnehmungen (35a) versehene längliche Röhrchen (36) im Profilquerschnitt ovalringförmig ausgebildet ist.

9. Implantat nach einem der Ansprüche 1 bis 8, **dadurch gekennzeichnet, dass** die mit dem Innenraum (35e) des Röhrchens (36) in Verbindung stehenden Öffnungen (35a) rechteckig, quadratisch oder trapezförmig ausgebildet und insbesondere gegenüberliegend zueinander angeordnet sind.

10. Implantat nach Anspruch 9, **dadurch gekennzeichnet, dass** die an der einen Seite vorgesehenen Öffnungen (35a) sowie die an der anderen gegenüberliegenden Seite des Röhrchens (36) vorgesehenen Öffnungen (35a') in axialer Richtung versetzt zueinander angeordnet sind.

11. Implantat nach einem der Ansprüche 1 bis 3, **dadurch gekennzeichnet, dass** die zwischen den einzelnen Öffnungen (35a) angeordneten stegförmigen Ringteile (35c) in axialer Richtung des Röhrchens (36) entweder in die eine oder in die andere Richtung geneigt sind.

12. Implantat nach einem der Ansprüche 1 bis 3, **dadurch gekennzeichnet, dass** die zwischen den einzelnen Öffnungen (35a) angeordneten stegförmigen Ringteile (35c) in axialer Richtung des Röhrchens (36) abwechselnd und gegenläufig geneigt zueinander angeordnet sind.

13. Implantat nach einem der Ansprüche 1 bis 12, **dadurch gekennzeichnet, dass** das biegsame Röhrchen (36) mindestens eine bis in halber Umfangsrichtung des freigelegten Schlemmschen Kanals (15) reichende Länge aufweist und am distalen Ende mit einem an der Sklera-Innenseite (13") anliegenden Bund (37) versehen ist.

14. Implantat nach einem der Ansprüche 1 bis 13, **dadurch gekennzeichnet, dass** das Röhrchen (36) aus biologisch verträglichem flexiblem Material, vorzugsweise aus polymerem Material mit thermisch- oder mechanischem Formgedächtniseffekt hergestellt ist.

15. Implantat nach einem der Ansprüche 1 bis 14, **dadurch gekennzeichnet, dass** das biegsame Röhrchen (36) aus biologisch verträglichem Material wie beispielsweise aus Gold oder Nitinol hergestellt ist.

## Claims

1. Implant for inserting into the Schlemm's canal (15) of an eye (10), composed of a flexible tube (36) extending in the direction of a longitudinal axis (Z), the tube (36) being insertable to at least a quarter of the circumferential direction of the lumen (16) of the circular Schlemm's canal (15), the tube (36) having several openings (35a) arranged at a distance from each other in the direction of the longitudinal axis (Z), **characterized in that** the tube (36) comprises, at the circumference, an axially extending connection part (35b), or two axially extending connection parts (35b) arranged diametrically to each other in relation to the longitudinal axis (Z), **in that** the tube (36) comprises several web-shaped ring parts (35c) arranged at a distance from each other in the axial direction and issuing from the connection part (35b), wherein the openings (35a) are arranged between the individual ring parts (35c), and wherein the openings (35a) form a connection to the interior (35e) of the tube (36), wherein each opening (35a) is arranged in the circumferential direction of the tube (36) between the connection part (35b) and in each case has an opening angle (W) oriented in the circumferential direction.

2. Implant according to Claim 1, **characterized in that** the tube (36) comprises two axially extending connection parts (35b), a first and a second connection part (35b), wherein the first and the second connection part (35b) are arranged opposite each other in relation to the longitudinal axis (Z) .

3. Implant according to Claim 2, **characterized in that** the openings (35a) provided between the first and the second connection part (35b), and also between the individual ring parts (35c) arranged at a distance from each other at the circumference of the tube (36), are each provided with an opening angle (W) of the order of between 90° and 105°.

4. Implant according to one of the preceding claims, **characterized in that** the ring parts (35c) extending between the connection part (35b) and arranged mutually at a distance from each other in the direction of the longitudinal axis (Z) extend perpendicularly with respect to the longitudinal axis (Z) and, between the ring parts (35c) at the circumference of the tube (36), form openings that each have circumferentially oriented recesses (35a) .

5. Implant according to Claim 1, **characterized in that** the recesses (35a) arranged at the circumference of the tube (36) are each provided with an opening angle (W) of the order of between 280° and 290°.

6. Implant according to Claim 1, **characterized in that** the web-shaped ring parts (35c) arranged at an axial distance from each other on the first connection part (35b) are each provided with a slit-shaped gap (35d) and in this way form two sub-sections, wherein the two sub-sections are expandable relative to each other.

7. Implant according to one of Claims 1 to 6, **characterized in that** the elongate tube (36), provided with the connection parts (35b) arranged parallel to each other, with the web-shaped ring parts (35c) and with the openings or recesses (35a) arranged therebetween, has a circular ringshaped profile cross section.

8. Implant according to one of Claims 1 to 6, **characterized in that** the elongate tube (36), provided with the connection parts (35b) arranged parallel to each other, with the web-shaped ring parts (35c) and with the openings or recesses (35a) arranged therebetween, has an oval ringshaped profile cross section.

9. Implant according to one of Claims 1 to 8, **characterized in that** the openings (35a) connected to the interior (35e) of the tube (36) have a rectangular, square or trapezoidal shape and in particular are arranged opposite each other.

10. Implant according to one of Claims 9, **characterized in that** the openings (35a) provided on one side of the tube (36) and the openings (35a') provided on the other and opposite side of the tube (36) are arranged offset in relation to each other in the axial direction.

11. Implant according to one of Claims 1 to 3, **characterized in that** the web-shaped ring parts (35c) arranged between the individual openings (35a) in the axial direction of the tube (36) are inclined either in one direction or the other.

12. Implant according to one of Claims 1 to 3, **characterized in that** the web-shaped ring parts (35c) arranged between the individual openings (35a) in the axial direction of the tube (36) are arranged inclined relative to each other alternately and in opposite directions.

13. Implant according to one of Claims 1 to 12, **characterized in that** the flexible tube (36) has at least a length reaching to one half of the circumference of the exposed Schlemm's canal (15) and is provided at the distal end with a collar (37) bearing on the inner aspect (13") of the sclera.

14. Implant according to one of Claims 1 to 13, **characterized in that** the tube (36) is made of biocompatible flexible material, preferably of polymer material having a thermal or mechanical shape-memory effect.

15. Implant according to one of Claims 1 to 14, **characterized in that** the flexible tube (36) is made of biocompatible material, for example gold or nitinol.

## Revendications

1. Implant destiné à être introduit dans le canal de Schlemm (15) d'un oeil (10), constitué d'un petit tube flexible (36) s'étendant dans la direction d'un axe longitudinal (Z), le petit tube (36) pouvant être inséré jusqu'à au moins un quart de la direction périphérique de la lumière (16) du canal de Schlemm circulaire (15), le petit tube (36) présentant plusieurs ouvertures (35a) qui sont disposées dans la direction de l'axe longitudinal (Z) à distance les unes des autres, **caractérisé en ce que** le petit tube (36) comprend, au niveau de la périphérie, une partie de liaison (35b) s'étendant dans la direction axiale ou deux parties de liaison (35b) s'étendant dans la direction axiale, diamétralement opposées l'une à l'autre par rapport à l'axe longitudinal (Z), **en ce que** le petit tube (36) comprend plusieurs parties annulaires en forme de nervures (35c) espacées les unes des autres dans la direction axiale, partant de la partie de liaison (35b), les ouvertures (35a) étant disposées entre les parties annulaires individuelles (35c), et les ouvertures (35a) constituant une liaison avec l'espace interne (35e) du petit tube (36), chaque ouverture (35a) étant disposée dans la direction périphérique du petit tube (36) entre la pièce de liaison (35b) et présentant à chaque fois un angle d'ouverture (W) orienté dans la direction périphérique.

2. Implant selon la revendication 1, **caractérisé en ce que** le petit tube (36) comprend deux parties de liaison (35b) s'étendant dans la direction axiale, une première et une deuxième partie de liaison (35b), la première et la deuxième partie de liaison (35b) étant disposées à l'opposé l'une de l'autre par rapport à l'axe longitudinal (Z).

3. Implant selon la revendication 2, **caractérisé en ce que** les ouvertures (35a) prévues à la périphérie du petit tube (36) entre la première et la deuxième partie de liaison (35b) et entre les parties annulaires individuelles (35c) disposées à distance les unes des autres sont à chaque fois pourvues d'un angle d'ouverture (W) d'un ordre de grandeur compris entre 90° et 105°.

4. Implant selon l'une quelconque des revendications précédentes, **caractérisé en ce que** les parties annulaires (35c) disposées à distance mutuelle les unes des autres dans la direction de l'axe longitudinal (Z), s'étendant entre la partie de liaison (35b), s'étendent perpendiculairement à l'axe longitudinal (Z), et constituent entre les parties annulaires (35c) au niveau de la périphérie du petit tube (36) des ouvertures avec des évidements (35a) orientés à chaque fois dans la direction périphérique.

5. Implant selon la revendication 1, **caractérisé en ce que** les évidements (35a) disposés à la périphérie du petit tube (36) sont chacun pourvus d'un angle d'ouverture (W) d'un ordre de grandeur compris entre 280° et 290°.

6. Implant selon la revendication 1, **caractérisé en ce que** les parties annulaires en forme de nervures (35c) disposées au niveau de la première partie de liaison (35b) à distance les unes des autres dans la direction axiale sont à chaque fois pourvues d'un interstice en forme de fente (35d) et constituent ainsi deux pièces partielles, les deux pièces partielles pouvant être écartées l'une de l'autre.

7. Implant selon l'une quelconque des revendications 1 à 6, **caractérisé en ce que** le petit tube allongé (36) pourvu des parties de liaison (35b) disposées parallèlement les unes aux autres, des parties annulaires en forme de nervures (35c) et des ouvertures ou des évidements (35a) disposés entre elles, est réalisé sous forme annulaire circulaire en section transversale de profilé.

8. Implant selon l'une quelconque des revendications 1 à 6, **caractérisé en ce que** le petit tube allongé (36) pourvu des parties de liaison (35b) disposées parallèlement les unes aux autres, des parties annulaires en forme de nervures (35c) et des ouvertures ou des évidements (35a) disposés entre elles, est réalisé sous forme annulaire ovale en section transversale de profilé.

9. Implant selon l'une quelconque des revendications 1 à 8, **caractérisé en ce que** les ouvertures (35a) en liaison avec l'espace interne (35e) du petit tube (36) sont réalisées sous forme rectangulaire, quadratique ou trapézoïdale et sont notamment disposées à l'opposé les unes des autres.

10. Implant selon l'une quelconque des revendications 9, **caractérisé en ce que** les ouvertures (35a) prévues au niveau d'un côté ainsi que les ouvertures (35a') prévues au niveau de l'autre côté opposé du petit tube (36) sont disposées de manière décalée les unes par rapport aux autres dans la direction axiale.

11. Implant selon l'une quelconque des revendications 1 à 3, **caractérisé en ce que** les parties annulaires (35c) en forme de nervures disposées entre les ouvertures individuelles (35a) sont inclinées dans la direction axiale du petit tube (36) dans l'une ou dans l'autre direction.

12. Implant selon l'une quelconque des revendications 1 à 3, **caractérisé en ce que** les parties annulaires (35c) en forme de nervures disposées entre les ouvertures individuelles (35a) sont disposées dans la direction axiale du petit tube (36) en alternance et avec une inclinaison opposée les unes par rapport aux autres.

13. Implant selon l'une quelconque des revendications 1 à 12, **caractérisé en ce que** le petit tube flexible (36) présente au moins une longueur s'étendant jusqu'à la moitié de la direction périphérique du canal de Schlemm exposé (15) et est pourvu à l'extrémité distale d'un épaulement (37) s'appliquant contre le côté inférieur de la sclère (13").

14. Implant selon l'une quelconque des revendications 1 à 13, **caractérisé en ce que** le petit tube (36) est fabriqué à partir de matériau flexible biologiquement compatible, de préférence à partir de matériau polymère avec un effet de mémoire de forme thermique ou mécanique.

15. Implant selon l'une quelconque des revendications 1 à 14, **caractérisé en ce que** le petit tube flexible (36) est fabriqué en matériau biologiquement compatible tel que par exemple en or ou en nitinol.
